(19) Europäisches Patentamt
European Patent Office
Office européen des brevets

(11) Publication number: 0 165 168
A2

# (12) EUROPEAN PATENT APPLICATION

(21) Application number: 85401091.5

(22) Date of filing: 04.06.85

(51) Int. Cl.[4]: C 07 C 67/36
C 07 C 69/76, C 07 C 51/10
C 07 C 63/06, C 07 C 102/00
C 07 C 103/76

(30) Priority: 06.06.84 US 617787

(43) Date of publication of application:
18.12.85 Bulletin 85/51

(84) Designated Contracting States:
DE FR GB IT

(71) Applicant: EASTMAN KODAK COMPANY
343 State Street
Rochester New York 14650(US)

(72) Inventor: Lentz, Carl Michael
P.O. Box 1972
Kingsport Tennessee 37662(US)

(72) Inventor: Overton, James Ray
P.O. Box 1972
Kingsport Tennessee 37662(US)

(72) Inventor: Cornell, David Dunlap
P.O. Box 1972
Kingsport Tennessee 37662(US)

(74) Representative: Parent, Yves et al,
Kodak-Pathé Département des Brevets et Licences 30,
rue des Vignerons B.P. 60
F-94302 Vincennes Cedex(FR)

## (54) Carbonylation process for the production of aromatic acids and derivatives thereof.

(57) The present invention provides a process for the preparation of aryl carboxylic acids and derivatives thereof by the carbonylation of diaryliodonium salts. The diaryliodonium salts are reacted with carbon monoxide and water of an alcohol or amine in the presence of a zero-valent metal catalyst consisting essentially of rhodium, ruthenium, and/or molybdenum.

Croydon Printing Company Ltd.

## Description

# Carbonylation Process for the Production of Aromatic Acids and Derivatives Thereof

The present invention provides a novel catalytic carbonylation process for the production of aromatic acids, esters, and/or amides. The novel process involves the carbonylation of a diaryliodonium salt in the presence of water, an alcohol, or an amine and a specified transition metal catalyst.

The preparation of carboxylic acid esters from organic halides by a carbonylation process has been described in U.S. Patent No. 3,988,358. ("the Heck process"). In the disclosed process, carboxylic acid esters or amides are obtained from aryl halides and substituted derivatives thereof by the reaction of the chosen starting material with an alcohol or primary or secondary amine and carbon monoxide in the presence of a palladium catalyst. A typical example is the conversion of bromobenzene to n-butyl benzoate at 100°C and one atmosphere of carbon monoxide in the presence of tri-n-butyl amine and a catalytic amount of $PdBr_2[P(C_6H_5)_3]_2$. This reaction does not involve the use of a diaryliodonium salt as a starting material.

Davidson et al (J. Chem. Soc. (A), 1968, pp. 1616-17) discloses the reaction of diphenyliodonium bromide and carbon monoxide at 190 atmospheres in methanol at 100°C in the absence of a catalyst. Methylbenzoate was produced in yields of about 35 to 50%.

It also is known that diaryliodonium salts can be carbonylated to aromatic esters and amides in the presence of a palladium catalyst. See, for example, Nippon Kagaku Kaishi, 1982, No. 2, pp. 236-241. The carbonylation reaction is described therein by the

following equation:

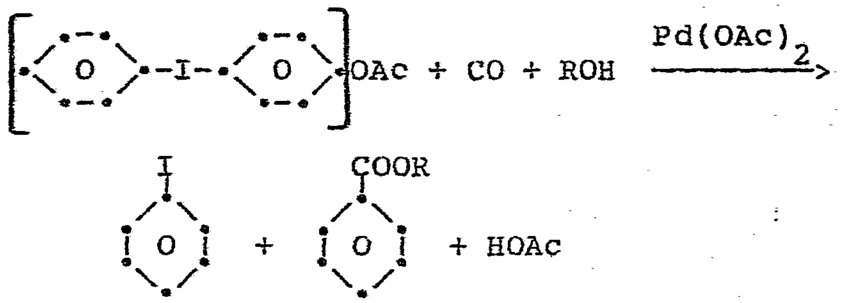


As indicated above, in addition to the desired ester product, there also is produced an aryl iodide (specifically, iodobenzene). This species can be reacted with another aryl compound (e.g., benzene, toluene, etc.) so as to produce the diaryliodonium salt which is employed as a starting material. However, the reference implicitly discloses that the aryl iodide by-product is carbonylated in the presence of palladium by the above-described Heck process. See, for example, Table II of the reference wherein 5.0 millimoles of diphenyliodonium bromide is converted at 50°C over a period of time of 60 minutes to 6.2 millimoles of methyl benzoate. Thus, both the diphenyliodonium bromide and the iodobenzene byproduct are involved in the formation of the methyl benzoate product. This side reaction is undesirable in that an otherwise recyclable by-product is at least partially consumed in the presence of a palladium catalyst. As a result, additional quantities of the appropriate (and relatively expensive) aryl iodide must be provided to the reaction system.

In contrast to these deficiencies of prior art processes, it has now been found that only the diaryliodonium salt is carbonylated to the desired aromatic acid or derivative thereof in the presence of specified catalytic species. The recyclable by-products thus are preserved for the regeneration of the diaryliodonium starting material.

## Summary of the Invention

The present invention provides a process for the preparation of compounds of the formula

$$Ar\overset{O}{\overset{\|}{C}}OR \quad \text{or} \quad Ar\overset{O}{\overset{\|}{C}}NHR' \quad ,$$

wherein R represents H or an aliphatic moiety having up to 12 carbon atoms and wherein R' represents an aliphatic moiety having up to 12 carbon atoms. The process comprises reacting a diaryliodonium salt of the formula

$$\begin{array}{c} Ar \\ | \\ I^{+}\ X^{-} \\ | \\ Ar' \end{array} \quad ,$$

wherein Ar and Ar' each independently represents a carbocyclic or heterocyclic aromatic moiety having 5 to 20 atoms in the ring or rings thereof and X represents a weak acid anion, with carbon monoxide and water or an aliphatic alcohol or primary amine having up to 12 carbon atoms in the presence of a zero-valent metal catalyst consisting essentially of rhodium, ruthenium, and/or molybdenum.

## Detailed Description of the Invention

The present invention relates to the production of aromatic carboxylic acids, esters, and/or amides by a process which involves the carbonylation of a diaryliodonium salt in the presence of a zero-valent metal catalyst consisting essentially of rhodium, ruthenium, and/or molybdenum.

The diaryliodonium salt employed as a starting material in the process of the present invention has the following chemical formula:

$$\begin{array}{c} Ar \\ | \\ I^{+}\ X^{-} \\ | \\ Ar' \end{array} \qquad (I)$$

In the above Formula I, Ar and Ar' each independently represents a carbocyclic or heterocyclic aromatic moiety having 5 to 20 atoms in the ring or rings thereof. Such moieties can be derived from, for example, toluene, benzene, naphthalene, pyridine, thiophene and pyrrole. Preferably, Ar and Ar' in the above formula represent the same aromatic moiety.

Furthermore, the aryl groups of the diaryliodonium salt can be connected by means of a carbon or heteroatom bridge, as exemplified by the following formulas:

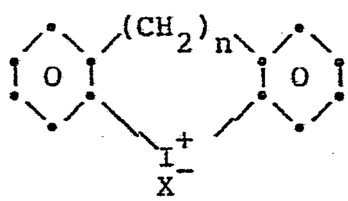 or 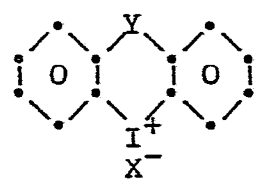

In the above formulas, n can equal 1, 2 or 3, and Y can represent O, S, or N.

The aromatic moieties of the diaryliodonium salt can be substituted or unsubstituted. When substituted, typical substituents include the halides, alkyl groups having up to 12 carbon atoms, vinyl, carboxylic acid moieties, carboxylic ester moieties, ether groups and nitro groups.

In the above Formula I, X represents a weak acid anion. Preferred examples of such anions include acetate, trihaloacetate (e.g., triiodoacetate), p-toluenesulfonate, benzenesulfonate, hydroxide, iodide, bromide and boron tetrafluoride.

Thus, preferred examples of the diaryliodonium salt include diphenyliodonium bromide, ditolyliodonium acetate, ditolyliodonium iodide, diphenyliodonium iodide and diphenyliodonium acetate.

The diaryliodonium salts employed in the process of the present invention can be prepared by the methods described by Beringer et al in J.A.C.S., 81, 342 (1959), the disclosure of which is incorporated herein by reference in its entirety.

In accordance with the process of the present invention, the diaryliodonium salt described above is reacted with water or an aliphatic alcohol or primary amine having up to 12 carbon atoms. In the case where water is employed as a reactant, an aromatic carboxylic acid is produced; when an alcohol is employed, the corresponding aromatic ester is produced; and when an amine is employed, the corresponding amide is produced.

In especially preferred embodiments of the present invention, aromatic esters are produced by the reaction of a diaryliodonium salt with an alcohol as described above. The aliphatic alcohol which is employed in the present process may be monofunctional or multifunctional. Thus, glycols and other polyols are suitable, as are glycol esters, glycol ethers, and other such derivatives. Preferably, the alcohol comprises a lower alkanol such as an alkanol having up to 12 carbon atoms, ethylene glycol, propylene glycol, neopentyl glycol, ethylene glycol monoacetate and mixtures thereof.

Suitable primary amines which may be employed in the preparation of amides by the process of the present invention include both aliphatic amines and aromatic amines. Suitable primary aliphatic amines include those having up to 12 carbon atoms, such as monoethylamine, mono-n-butylamine, mono-2-ethylhexylamine, octylamine and dodecylamine. Suitable aromatic amines include aniline and substituted anilines. Multifunctional primary amines, such as the phenylenediamines, also may be used in the present process.

The reaction system of the present process further comprises a zero-valent metal catalyst. The catalyst consists essentially of a metal selected from the group consisting of rhodium, ruthenium, and/or molybdenum. Rhodium is the preferred metal catalyst.

The active metal species is the zero-valent form of the metal. Therefore, in preferred embodiments, the catalyst is provided to the reaction system in the zero-valent form of the metal. More preferably, the zero-valent metal is supported on a suitable material. For example, a highly desirable catalyst material comprises 5% rhodium on a carbon support. Of course, other zero-valent catalyst forms can be employed such as the metal carbonyls, such as molybdenum hexacarbonyl.

The catalyst metal may also be provided in a higher valence state, provided that an in situ reduction to the zero-valent form occurs. Thus, rhodium triacetate and rhodium trichloride also are suitable catalyst materials.

The catalyst is present in a concentration of at least 0.01 millimole per mole of iodonium salt, preferably, 0.1 to 1 millimole per mole.

The catalytic carbonylation reaction of the present invention is conducted in the presence of carbon monoxide, which is employed in amounts such that the total reaction pressure is in the range of atmospheric to 500 psig. Superatmospheric pressure may be advantageous when a volatile reactant is employed or when an increase in the rate of reaction is desirable. Thus, reaction pressures from atmospheric pressure up to 3500 kPa are suitable, with pressures from atmospheric pressure up to 800 kPa being preferred.

The process of the present invention can be conducted at room temperature or at elevated temperatures up to 150°C. Preferably, the temperature of the reaction is in the range of 25 to 75°C.

It may be desirable also to include in the reaction system a base having a $pK_a$ greater than that of pyridine. The presence of such a base may aid

in the prevention of deactivation of the metal catalyst. When employed, the base is present in an amount of 1 to 5 equivalents of base per equivalent of catalyst. Preferably, 3 equivalents of base per equivalent of catalyst is employed. Preferred bases include trialkylamines, such as triethylamine and sodium carbonate.

Inert coordinating solvents may be employed, but are not necessary. Such solvents may include, for example, tetrahydrofuran and acetonitrile. In those aspects of the present invention wherein aromatic esters are produced by the reaction of a diaryliodonium salt with carbon monoxide and an alcohol, the alcohol can be employed as solvent. Likewise, when preparing amides, the amine can be employed as solvent.

While not wishing to be bound by theoretical considerations, it is believed that the process of the present invention involves the following reaction pathway:

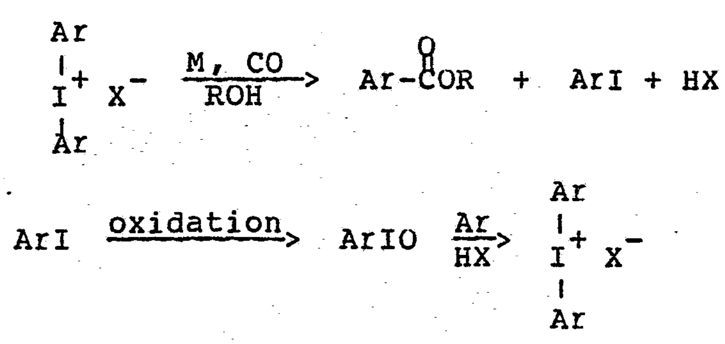


This general procedure can be more specifically exemplified in the case where Ar represents toluene, M represents Rh, HX represents acetic acid (HOAc), and

ROH represents methanol, as follows:

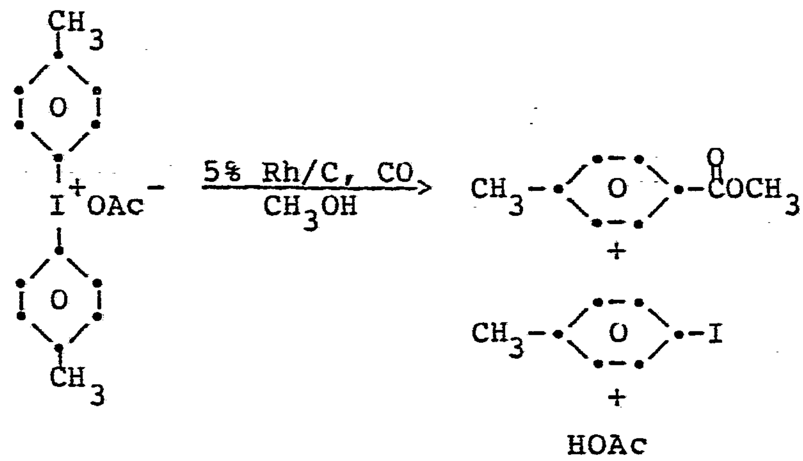


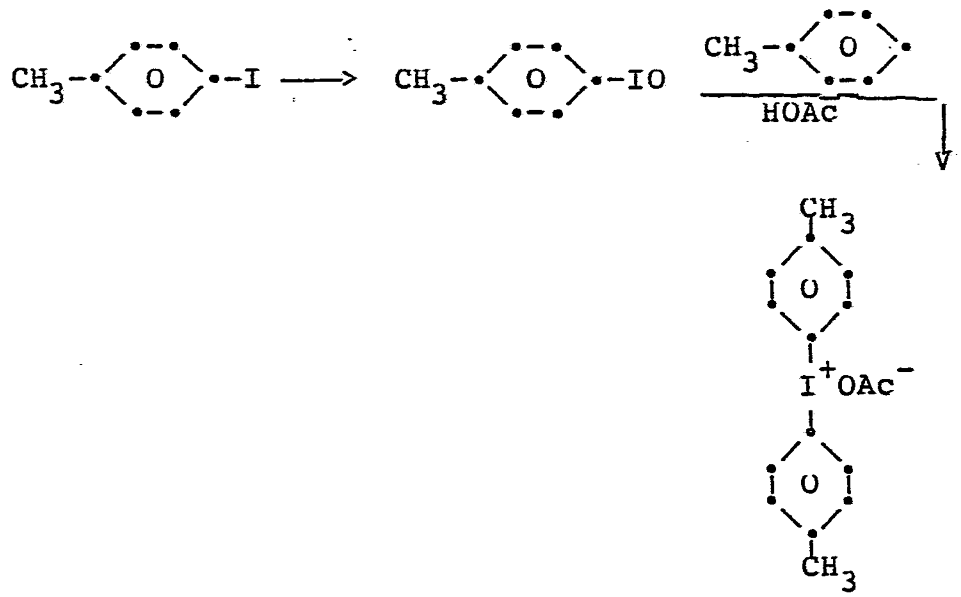


In the above-described reaction scheme, the aryl iodide by-product can be removed from the reaction system by distillation and then employed in the preparation of the diaryliodonium salt starting material. In contrast to prior art processes, the aryl iodide in the present process is not consumed by carbonylated to an aromatic acid or derivative thereof and is therefore available for use in the preparation of the diaryliodonium salt starting material. This improvement represents an economic advantage in that the iodide-containing product is preserved and

recycled. In this manner, this relatively expensive component of the reaction system need not be supplied to the reaction process in considerable quantities on a continuous basis, as was necessary with prior art processes.

The process of the present invention provides products which are useful as intermediates in the synthesis of polyesters such as polyethylene terephthalate, and other useful polymeric materials.

The invention will be further illustrated by the following Examples although it will be understood that these Examples are included merely for purposes of illustration and are not intended to limit the scope of the invention.

EXAMPLES 1-10

The following Examples illustrate the carbonylation of a ditolyliodonium salt in the presence of an alcohol, a zero-valent metal catalyst, and carbon monoxide so as to produce the corresponding p-methylbenzoate ester and iodotoluene. The particular anion, alcohol, catalyst, and base, if present, employed in each example are indicated below in Table I. The catalyst was employed in a concentration of 0.05 millimole per mole of iodonium salt. The base, if employed, was present in a concentration of 3.0 millimoles per millimole of iodonium salt.

In each Example, a 100 ml three-neck, round bottom flask was fitted with a reflux condenser, a thermometer, magnetic stirrer, and a gas dispersion tube. To the described apparatus were added 1.0 millimoles of the indicated diaryliodonium salt, 0.10 g of the indicated catalyst, 3.0 millimoles of the indicated base, and 50 ml of the indicated alcohol. While carbon monoxide was fed beneath the surface of the reaction mixture at the indicated total pressure, the

reaction mixture was heated to the indicated temperature and was held at that temperature for the indicated period of time. The resulting mixture was cooled to 25 to 30°C and was filtered through a Celite pad. Water (250 ml) was added to the filtrate, and the resulting mixture was extracted three times with 50 ml of diethyl ether. The ether extracts were combined and dried over magnesium sulfate. The solvent was removed in vacuo to yield a light yellow oil. GLPC analysis, employing authentic samples for comparisons, indicated the presence of p-methyltoluate (p-2-hydroxyethyltoluate in Example 5) and p-iodotoluene as the only products of reaction.

The results of these Examples are given below in Table I.

TABLE I

| Ex. | X | Alcohol | Catalyst | Temperature (°C) | Pressure (psig) | Base | Conversion (%) | Time (hrs) |
|---|---|---|---|---|---|---|---|---|
| 1 | $OAc^-$ | Methanol | 5% Rh/C | 50 | Atm | - | 91 | 2 |
| 2 | $OAc^-$ | Methanol | 5% Rh/C | 50 | Atm | - | 91 | 6 |
| 3 | $OAc^-$ | Methanol | 5% Rh/C | 80 | 100 | - | 48 | 1 |
| 4 | $OAc^-$ | Methanol | 5% Rh/C | 80 | 100 | - | 47 | 1 |
| 5 | $I^-$ | Ethylene Glycol | 5% Rh/C | 80 | 100 | $Et_3N$ | 48 | 2 |
| 6 | $I^-$ | Methanol | 5% Rh/C | 80 | 100 | $Et_3N$ | 85 | 1 |
| 7 | $I^-$ | Methanol | 5% Rh/C | 80 | 100 | $Et_3N$ | 78 | 1 |
| 8 | $BF_4^-$ | Methanol | 5% Rh/C | 50 | Atm | $Et_3N$ | 97 | 3 |
| 9 | $I^-$ | Methanol | $Mo(CO)_6$ | 50 | Atm | $Et_3N$ | 22 | 2 |
| 10 | $I^-$ | Methanol | 5% Ru/C | 50 | Atm | $Et_3N$ | 9 | 2 |

COMPARATIVE EXAMPLES 1-4

These Comparative Examples demonstrate the advantages of the present invention. In particular, these Compartive Examples demonstrate that the iodotoluene by-product of the above Examples is carbonylated in the presence of a palladium catalyst, but is preserved for recycling in the presence of a rhodium catalyst.

In each of the following Comparative Examples, 1.0 millimole of p-iodotoluene, 0.10 g of the indicated catalyst, 3.0 millimole of triethylamine, and 40 ml of the indicated alcohol were provided to the above-described reaction apparatus. While carbon monoxide was fed beneath the surface of the reaction mixture so that the indicated total pressure was obtained, the reaction mixture was heated to the indicated temperature and was held at that temperature for the indicated period of time. Upon completion of reaction, the reaction mixture was worked up as described above. The results are given below in Table II.

TABLE II

| Comp. Ex. | Alcohol | Catalyst | Temperature (°C) | Pressure (psig) | Conversion (%) | Time (hrs) |
|---|---|---|---|---|---|---|
| 1 | Methanol | 5% Rh/C | 80 | 100 | 0 | 2 |
| 2 | Ethylene Glycol | 5% Rh/C | 80 | 100 | 0 | 2 |
| 3 | Methanol | 5% Pd/C | 50 | Atm | 11.7 | 2 |
| 4 | Ethylene Glycol | 5% Pd/C | 80 | 100 | 71.7 | 1.5 |

These results clearly demonstrate that conversion of the p-iodotoluene substrate to the corresponding ester occurred in the presence of the palladium catalyst, while no reaction occurred in the presence of rhodium catalyst. Thus, in the overall process of the present invention, the p-iodotoluene by-product is not consumed but is available to be recycled, thereby enhancing the economics of the reaction.

The invention has been described in detail with particular reference to preferred embodiments thereof, but it will be understood that variations and modification can be effected within the spirit and scope of the invention.

We Claim:

1. A process for the preparation of compounds of the formula

$$Ar\overset{O}{\overset{\|}{C}}OR \quad \text{or} \quad Ar\overset{O}{\overset{\|}{C}}NHR' ,$$

wherein R represents H or an aliphatic moiety having up to 12 carbon atoms and R' represents an aliphatic moiety having up to 12 carbon atoms, said process comprising reacting a diaryliodonium salt of the formula

$$\begin{matrix} Ar \\ | \\ I^{+} \\ | \\ Ar' \end{matrix} \; X^{-} \quad ,$$

wherein Ar and Ar' each independently represents a carbocyclic or heterocyclic aromatic moiety having 5 to 20 atoms in the ring or rings thereof and X represents a weak acid anion,

with (i) carbon monoxide and (ii) water or an aliphatic alcohol or primary amine having up to 12 carbon atoms in the presence of a zero-valent metal catalyst consisting essentially of rhodium, ruthenium, and/or molybdenum.

2. The process of Claim 1 wherein Ar and Ar' represent moieties derived from toluene, benzene, naphthalene, pyridine, thiophene, or pyrrole.

3. The process of Claim 2 wherein $X^{-}$ represents acetate, trihaloacetate, p-toluenesulfonate, benzenesulfonate, hydroxide, iodide, bromide, or boron tetrafluoride.

4. The process of Claim 3 wherein said alcohol comprises a lower alkanol, ethylene glycol, propylene glycol, neopentyl glycol, ethylene glycol monoacetate, or a mixture thereof.

5. The process of Claim 4 wherein said catalyst consists essentially of rhodium.

6. A process for the preparation of aromatic esters which comprises reacting at a temperature of 25 to 150°C a diaryliodonium salt of the formula

Ar
|
$I^{+} X^{-}$ ,
|
Ar

wherein Ar represents a moiety derived from toluene, benzene, naphthalene, pyridine, thiophene, or pyrrole and X represents a weak acid anion comprising acetate, trihalo-acetate, p-toluenesulfonate, benzene-sulfonate, hydroxide, iodide, bromide, or boron tetrafluoride,

with carbon monoxide and an alcohol comprising a lower alkanol, ethylene glycol, propylene glycol, neopentyl glycol, ethylene glycol monoacetate, or a mixture thereof in the presence of a zero-valent rhodium catalyst and a base having a $pK_a$ greater than that of pyridine.